# EUROPÄISCHE PATENTSCHRIFT

(11) **EP 0 362 728 B1**
(45) Veröffentlichungstag und Bekanntmachung des Hinweises auf die Patenterteilung: **23.11.1994**
(21) Anmeldenummer: 89118140.6
(22) Anmeldetag: 30.09.1989
(51) Int. Cl.: C07C 57/30, C07C 51/43, A61K 31/19, A61K 9/16

(54) **Verfahren zur Gewinnung von Ibuprofen für die Direkttablettierung**
Process for preparing ibuprofen for direct tabletting
Procédé de préparation d'une ibuprofène pour compression directe

(30) Priorität: 01.10.1988 DE 3833448
(43) Veröffentlichungstag der Anmeldung: 11.04.1990
(73) Patentinhaber: HOECHST AKTIENGESELLSCHAFT, 65926 Frankfurt am Main (DE); HOECHST CELANESE CORPORATION, Somerville, N.J. 08876 (US)
(72) Erfinder: Rittner, Siegbert, Dr., D-6082 Mörfelden-Walldorf (DE); Stüven, Uwe, D-6232 Bad Soden am Taunus (DE); Steidl, Dieter, D-6238 Hofheim am Taunus (DE); Sickmüller, Alfred, Dr., D-6000 Frankfurt am Main 70 (DE); Wheeler, Larry O. Dr., Corpus Christi TX 78410 (US); Moss, Gary L., Corpus Christi TX 78410 (US); Zey, Edward G. Dr., Corpus Christi TX 78412 (US)
(74) Vertreter: Fischer, Hans-Jürgen, Dr.

(56) Entgegenhaltungen:
- EP-A- 0 326 027
- FR-A- 2 253 508
- GB-A- 2 187 952
- JP-A-81 120 616
- PATENT ABSTRACTS OF JAPAN, Band 5, Nr. 200 (C-84)[872], 18. Dezember 1981;& JP-A-56 120 616 (KAKEN YAKUKAHOU) 22-09-1981
- IDEM

## Beschreibung

Die vorliegende Erfindung betrifft ein Verfahren zur Gewinnung von gut rieselfähigem Ibuprofenpulver mit verbesserten Lager- und Formulierungseigenschaften für die Direkttablettierung. Das genannte Ibuprofen (2-(4-Isobutylphenyl)-propionsäure) findet vielfältige Verwendung, z. B. als Analgeticum, Antipyreticum oder Antirheumaticum.

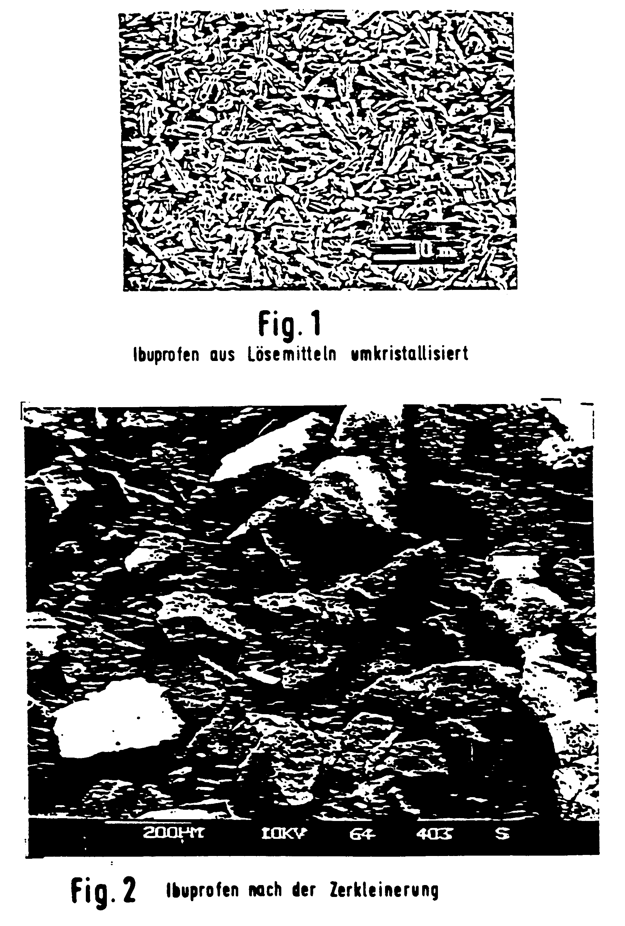

Nach dem heutigen Stand der Technik wird das auf den verschiedenen Reaktionswegen gewonnene Arzneimittel vor der Trocknung und Formulierung aus verschiedenen Lösemitteln umkristallisiert. Bei den Umkristallisationsprozessen, die aus einer Reihe von Lösemitteln, wie in den Patentschriften GB 971700, dem EP-A 170147 oder der rumänischen Anmeldung 79345 beschrieben, durchgeführt werden können, werden stets Kristalle mit nadelförmigem Habitus erhalten. Die so z. B. aus Wasser/Methanol, n-Hexan, oder n-Heptan gewonnen nadelförmigen getrockneten Kristalle (siehe Fig. 1), haben jedoch sowohl bei der Handhabung, der Lagerung wie auch bei der Herstellung von pharmazeutischen Zübereitungsformen eine Reihe von gravierenden Nachteilen. Bei der Trocknung nach Umkristallisation laden sich die Kristalle statisch auf, was ihre Handhabung erschwert. Weiterhin haben sie schlechte Fließ-, Riesel- und Lagereigenschaften und erleiden leicht einen Kristallbruch, was zur Staubbildung und zum Zusammenbacken des Produkts führt. Aus diesen Gründen bereitet das nadelförmige Ibuprofen Probleme bei der Verarbeitung mit entsprechenden Zuschlagstoffen (Hilfsstoffen). Zum Beispiel müssen für die Tablettierung relativ große Mengen an Zuschlagstoffen verwendet werden (ca. 50 % bezogen auf den Wirkstoff), was in Anbetracht der hohen Dosierung des Wirkstoffes zu großen Tabletten führt, was wiederum die Schluckbarkeit und damit die "Patientencompliance" negativ beeinflussen kann.

Man kann diese Nachteile überwinden, wenn man zur Konfektionierung anstelle eines aus Lösemitteln umkristallisierten (nadelförmigen) Produkts von einer Schmelze ausgeht.

Das japanische Patent Kokai 81-120616 mit dem Titel "Ibuprofen-Containing Granules, Production Method Thereof, and Internal Medicines Containing the Granules" beschreibt die Herstellung von Ibuprofen-Körnchen. Das geschmolzene Material wird auf Pergamentpapier gegossen und man läßt bei Raumtemperatur abkühlen. Eine Verfestigung des Materials tritt nach etwa 20 Minuten ein. Danach wird der erhaltene Feststoff mittels einer Walze, einer Hammermühle oder einer Siebschlagmühle zerkleinert, so daß ein Pulver erhalten wird.

Die Verfestigung einer Ibuprofen-Schmelze ist außerordentlich problematisch, da die bei etwa 74,8 °C erstarrende Ibuprofenschmelze beim Abkühlen ölig-viskos wird, zur Unterkühlung neigt und nur sehr langsam in den kristallinen Zustand überführt werden kann.

Es wurde überraschenderweise gefunden, daß man diese Nachteile überwinden kann, wenn die Ibuprofen -Schmelze unter spezifischen Bedingungen auf einem Walzen- oder Bandkühlapparat bei einer Temperatur von 0 - 50 °C, vorzugsweise bei einer Temperatur von 0 - 30 °C in kurzer Zeit zum Erstarren bringt und und bei der Verfestigung der Ibuprofen-Schmelze Impfgut verwendet und die dabei entstandenen Schuppen, Schülpen oder ähnlichen Gebilde unter speziellen Mahlbedingungen zerkleinert. Hierbei werden wie in Fig. 2 gezeigt, rundliche Feststoffpartikeln mit ausgezeichneten Riese-, Lager- und Konfektionier-Eigenschaften gewonnen, die sich nicht statisch aufladen, leicht handhabbar sind und sich für die Direkttablettierung eignen.

Erfindungsgegenstand ist somit ein Verfahren zur Gewinnung von Ibuprofen-Partikeln durch Verfestigung einer Ibuprofen-Schmelze auf einem Kontaktkühlapparat und nachfolgende Zerkleinerung, dadurch gekennzeichnet, daß die Verfestigung der Ibuprofen-Schmelze auf einem Walzen- oder Bandkühlapparat bei einer Temperatur von 0 - 50° C, vorzugsweise bei einer Temperatur von 0 - 30° C, unter Benutzung von Impfgut erfolgt.

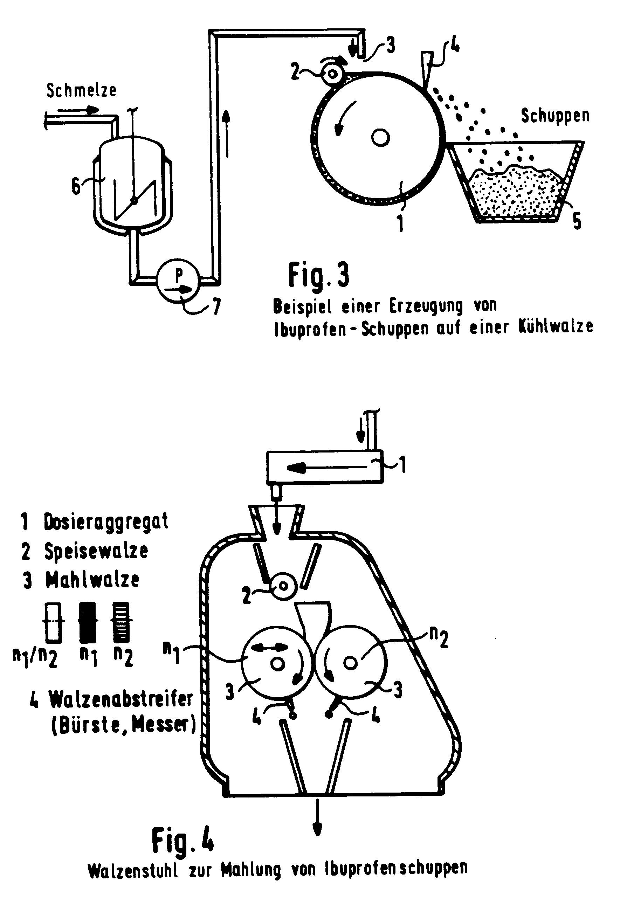

Die Verfestigung der Schmelze kann wie bereits erwähnt auf einem Walzen-(siehe Fig. 3) oder Bandkühlapparat erfolgen (vgl. z.B. Kristallisation aus Schmelzen, G. Matz: CIT 52 (1980) 7,570-575; Anwendung und Bauformen der Kühlwalze, M. Preger: Aufber. tech. 9 (1970), 551-558).

Das Impfgut kann beispielsweise durch Aufzug einer primär auf dem Kontaktkühlapparat erstarrenden Schmelzschicht erfolgen. Diese Schmelzschicht sollte eine Stärke von 0,1 bis 1,0 mm, vorzugsweise bis maximal 0,5 mm, aufweisen. Weiterhin kann das Impfgut in Partikelform auf die Walze gebracht werden oder in die Schmelze eingearbeitet werden. Falls das Impfgut in die Schmelze eingearbeitet wird ist eine Menge von vorzugsweise 10 bis 50 Gew.-%, bezogen auf die Schmelze, besonders bevorzugt von bis zu 35 Gew.-% zu wählen. Als Impfgut kommen insbesondere übliche Hilfsstoffe für die Arzneimittelherstellung sowie Ibuprofen-Partikeln oder Mischungen dieser Stoffe in Frage.
Die Temperatur der zur Anwendung kommenden Kühlfläche sollte vorzugsweise zwischen 0° und 50°C, besonders bevorzugt im Bereich von 10 bis 30°C liegen.
Je nach Kühlapparatetyp und Animpfungsmethode kann die Verweilzeit des Ibuprofens auf dem Kühlapparat zwischen ca. 25 s und 60 s liegen. Bei Aufbringen einer primär erstarrenden Schicht auf einer Kühlwalze erreicht man Verweilzeiten zwischen 5 und 10 s. Die dabei erzeugten Schuppen fallen mit einem Schüttgewicht zwischen 200 g/l und 400 g/l für die anschließende Zerkleinerung an. Die spezifische Leistung beträgt zwischen 10 kg/m²h und 120 kg/m²h erstarrter Schmelze.
Beim nachfolgenden Zerkleinerungsprozeß kommt es darauf an, Ibuprofen-Partikeln mit einer bestimmten Größe und einem möglichst engen Größenverteilungsspektrum zu erhalten. Zu bevorzugen ist eine mittlere Korngröße der Ibuprofen-Partikeln zwischen ca. 20µm und 200 µm. Zur Zerkleinerung des Ibuprofens können unterschiedliche Maschinen zur Anwendung kommen. Besonders geeignet ist für den Zerkleinerungsprozeß eine Zerkleinerungsmaschine, die mit zwei Walzen arbeitet, d. h. eine sogenannter Walzenstuhl (siehe Fig. 4, vgl. z.B. Grundriß der chemischen Technik, Verlag Chemie, Weinheim/Bergstraße 1968, S.33 J.) Mit einer derartigen Maschine läßt sich durch die exakte Festlegung der Mahlspaltes (Abstand zwischen zwei Walzen), des Drehzahlverhältnisses und der Walzenoberfläche der Grad der Zerkleinerung besonders präzise einstellen.

Für das erfindungsgemäße Verfahren zur Herstellung von Ibuprofen-Partikeln können prinzipiell beliebige Ibuprofen-Schmelzen verwendet werden. Besondere Bedeutung besitzt das Verfahren allerdings für Ibuprofen-Schmelzen, die bei einer Rektifikation des Ibuprofens z.B. gemäß der deutschen Patentanmeldung P 38 02 619 anfallen. Das wie zuletzt beschrieben rektifizierte Ibuprofen kann unmittelbar der Verfestigung auf einem Kühlapparat unterworfen werden, wodurch eine ökonomische Darstellungsmethode für hochwertige Ibuprofen-Partikeln gegeben ist, unter Meidung von Arbeitsschritten mit Beteiligung von Lösemitteln mit allen bekannten Nachteilen wie das Anfallen von Lösemittelabfällen, Lösemittelabluft etc.. Die erfindungsgemäß hergestellten Ibuprofen-Partikeln können in bekannter Weise zu Arzneimitteln in unterschiedlicher Darreichungsform weiterverarbeitet werden, wobei die Weiterverarbeitung im Vergleich zum Stand der Technik vereinfacht ist und mit weniger Hilfstoff durchgeführt werden kann.

Durch die nachfolgenden Beispiele, die im einzelnen
- die Erstarrung der Schmelze zu Schuppen,
- den anschließenden Mahlprozeß sowie schließlich
- die Direkttablettierung umfaßt, soll die Erfindung näher erläutert werden.

### 1. Beispiel zur Schuppenbildung

Die Ibuprofenschmelze wird aus der Vorlage 6, (vgl. Fig. 3) bei etwa 80 °C bis 90 °C mit der Pumpe 7 zur Schmelzeaufgabe 3 gefördert. Zwischen der Auftragswalze 2 und der Kühlwalze 1 bildet sich ein Sumpf aus. Durch Einstellen der wie die Kühlwalze auf beispielsweise 15 °C temperierten Auftragswalze wird eine Schichtdicke von z. B. 1 mm gewählt. Da das Abnahmemesser 4 in diesem Beispiel eine Restschicht von 0,5 mm auf der Walze zurückläßt, wird eine primäre Schicht zur Animpfung der frischen Schmelze zur Verfügung gestellt. Bei 6 s Verweilzeit führt die erläuterte Betriebsweise zu einer flächenspezifischen Leistung von 100 kg/m²h an geschuppter Schmelze.

### 2. Beispiel zur Zerkleinerung der Schuppen

Die nach Beispiel 1 gewonnenen Ibuprofenschuppen wurden mit Hilfe eines Walzenstuhls (vgl. Abb. 4) der folgenden Abmessungen unter den nachstehenden Bedingungen zerkleinert

| | |
|---|---|
| - Walzendurchmesser: | 250 mm |
| - Walzenbreite: | 100 mm |
| - Mahlspalt 1. Stufe bei Verwendung von Riffelwalzen mit 5 Riffeln pro cm: | 0,1 mm |
| - Mahlspalt 2. Stufe mit Glattwalzen: | 0,1 mm |
| - Mahlgut-Temperatur: | 25 °C |
| - Produkt-Durchsatz: | 25 kg/h |

Die Kornverteilung des Mahlguts, das für die Direkt-Tablettierung verwendet wurde, zeigt Tabelle 1.

**Tabelle 1**

| **Kornanalyse von gemahlenem Ibuprofen** | | |
|---|---|---|
| d (µm) | % Fraktion | % Rueckstand |
| > 400 | 0,40 | 0,40 |
| 400-300 | 1,90 | 2,30 |
| 300-250 | 5,00 | 7,30 |
| 250-200 | 19,20 | 26,50 |
| 200-150 | 28,30 | 54,80 |
| 150-125 | 13,50 | 68,30 |
| 125-90 | 10,40 | 78,70 |
| 90-60 | 5,80 | 84,50 |
| 60-40 | 6,00 | 90,50 |
| < 40 | 9,50 | 100,00 |

### 3. Anwendungsbeispiel

Ibuprofen-Tabletten bzw. überzogene Tabletten zu 200 mg Zusammensetzung.

| | **1 Tablette** |
|---|---|
| 1.) Ibuprofenpulver, gewonnen nach Beispiel 1 und 2: | 200 mg |
| 2.) Mikrokristalline Cellulose: | 40,5 mg |
| 3.) Natriumstärkeglykolat: | 2,0 mg |
| 4.) Hochdisperses Siliciumdioxid: | 1,5 mg |
| 5.) Magnesiumstearat: | 1,0 mg |

### Herstellung

Die Substanzen 1.) - 5.) werden in einem geeigneten Mischer gemischt und auf einer Rundläufer-Tabletten-Maschine zu Tabletten verpreßt. Falls erwünscht, können diese Tabletten in bekannter Weise mit Filmlacklösungen oder -suspensionen auf wäßriger bzw. organischer Basis, z. B. mit Celluloseethern als Filmbildnern überzogen werden. Ebenfalls ist ein Überziehen mit Drageesuspensionen auf Saccharosebasis möglich.

Die auf diese Weise hergestellten Tabletten hatten die folgenden Eigenschaften:

| | |
|---|---|
| - Härte: | 70 N |
| - Abrieb: | 0,5 % |
| - Zerfallszeit: | < 1 Min. |

### In vitro Wirkstoff-Freigabe (dissolution rate)

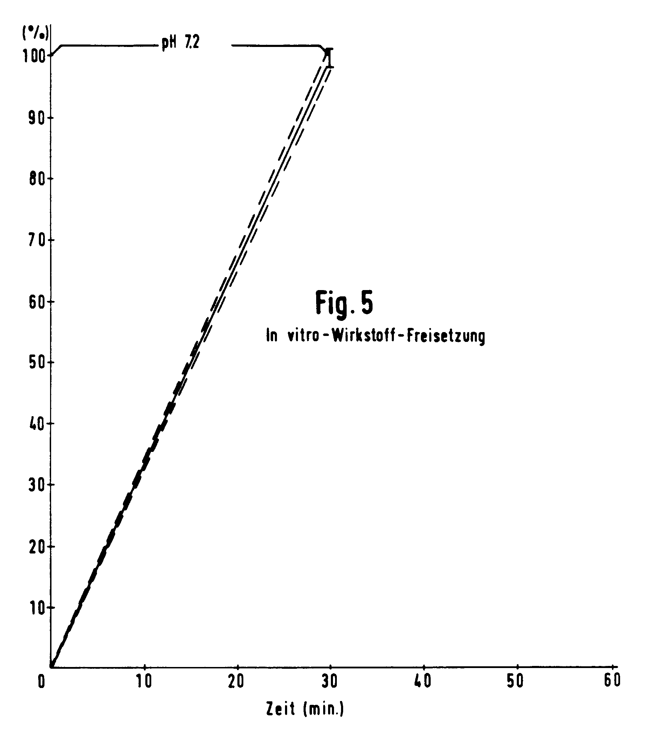

Die "dissolution rate" von Ibuprofen-Tabletten zu 200 mg wurde gemäß den Anforderungen der USP XXI nach folgender Methode bestimmt:
Apparatur 1 (Drehkörbchen-Methode), pH 7,2, Phosphatpuffer, 900 ml, 150 Upm, Prüfdauer 30 Min. Limit nach USP XXI Q = 50 %, Ergebnis 100 % (vgl. Fig. 5)

## Patentansprüche

1. Verfahren zur Gewinnung von Ibuprofen-Partikeln durch Verfestigung einer Ibuprofen-Schmelze auf einem Kontaktkühlapparat und nachfolgende Zerkleinerung, dadurch gekennzeichnet, daß die Verfestigung der Ibuprofen-Schmelze auf einem Walzen- oder Bandkühlapparat bei einer Temperatur von 0 - 50° C, vorzugsweise bei einer Temperatur von 0 - 30° C, unter Benutzung von Impfgut erfolgt.

2. Verfahren nach Anspruch 1, dadurch gekennzeichnet, daß als Impfgut Hilfsstoffe verwendet werden.

3. Verfahren nach Anspruch 1, dadurch gekennzeichnet, daß als Impfgut Ibuprofen-Partikeln verwendet werden.

4. Verfahren nach einem oder mehreren der Ansprüche 1-3, dadurch gekennzeichnet, daß zur Zerkleinerung des Ibuprofens ein Walzenstuhl benutzt wird.

5. Verfahren nach einem oder mehreren der Ansprüche 1-4, dadurch gekennzeichnet, daß das Ibuprofen für die Ibuprofen-Schmelze durch eine Rektifikation gereinigt worden ist.

## Claims

1. A process for the isolation of ibuprofen particles by solidifying an ibuprofen melt on a contact cooling apparatus and then comminuting the solid, wherein the solidification of the ibuprofen melt is carried out on a roll or belt cooling apparatus at a temperature of 0-50°C, preferably at a temperature of 0-30°C, using seed material.

2. The process as claimed in claim 1, wherein auxiliaries are used as the seed material.

3. The process as claimed in claim 1, wherein ibuprofen particles are used as the seed material.

4. The process as claimed in one or more of claims 1-3, wherein a roll mill is used for comminution of the ibuprofen.

5. The process as claimed in one or more of claims 1-4, wherein the ibuprofen for the ibuprofen melt has been purified by rectification.

## Revendications

1. Procédé d'obtention de particules d'ibuprofène par solidification d'ibuprofène fondu sur un dispositif refroidisseur contact, suivie de pur procédé caractérisé en ce que la solidification de la masse d'ibuprofène fondu est réalisée sur un dispositif refroidisseur à cylindre ou à bande à une température de 0 à 50°C, de préférence de 0 à 30°C, et à l'aide de matériau d'ensemencement.

2. Procédé selon la revendication 1, caractérisé en ce qu'on utilise des adjuvants comme matériaux d'ensemencement.

3. Procédé selon la revendication 1, caractérisé en ce qu'on utilise des particules d'ibuprofène comme matériaux d'ensemencement.

4. Procédé selon une ou plusieurs des revendications 1 à 3, caractérisé en ce qu'on utilise un moulin à cylindres pour le broyage de l'ibuprofène.

5. Procédé selon une ou plusieurs des revendications 1 à 4, caractérisé en ce que l'ibuprofène destiné à constituer la masse fondue d'ibuprofène a été purifié par une rectification.
